# EUROPÄISCHE PATENTSCHRIFT

(11) **EP 1 113 795 B1**
(45) Veröffentlichungstag und Bekanntmachung des Hinweises auf die Patenterteilung: **13.07.2005**
(21) Anmeldenummer: 99947352.3
(22) Anmeldetag: 15.09.1999
(51) Int. Cl.: A61K 31/405, A61P 35/00, A61P 25/00, A61P 25/28, A61P 27/12, A61P 9/04, A61P 9/10

(54) **TRYPTOPHANYLESTER UND DEREN N-ACYL-DERIVATE ZUR PRÄVENTION UND THERAPIE VON ERKRANKUNGEN, DIE DURCH OXIDATIONSPROZESSE VERURSACHT ODER VERSTÄRKT WERDEN**
TRYPTOPHANYL ESTERS AND THEIR N-ACYL DERIVATIVES FOR THE PREVENTION AND TREATMENT OF DISEASES CAUSED OR EXACERBATED BY OXIDATION PROCESSES
ESTERS DE TRYPTOPHANE ET LEURS DERIVES N-ACYLE DESTINES A LA PREVENTION ET AU TRAITEMENT DE MALADIES DUES AU PROCEDE D'OXYDATION OU AMPLIFIEES PAR CE DERNIER

(30) Priorität: 16.09.1998 DE 19842416
(43) Veröffentlichungstag der Anmeldung: 11.07.2001
(73) Patentinhaber: Max-Planck-Gesellschaft zur Förderung der Wissenschaften e.V., Berlin (DE)
(72) Erfinder: BEHL, Christian, D-80933 München (DE); MOOSMANN, Bernd, D-81547 München (DE)
(74) Vertreter: VOSSIUS & PARTNER
(86) Internationale Anmeldenummer: PCT/EP1999/006819
(87) Internationale Veröffentlichungsnummer: WO 2000/015206

(56) Entgegenhaltungen:
- EP-A- 0 041 935
- EP-A- 0 240 352
- WO-A-99/06393
- US-A- 4 448 785
- US-A- 5 599 947
- NAKAMIYA, TERUAKI ET AL: "Antibacterial activity of lauryl ester of DL-lysine" HAKKO KOGAKU ZASSHI (1976), 54(6), 369-73 , XP000910389
- RAO, KANDUKURI S. P. BHUSHANA ET AL: "Vinblastin-23-oyl amino acid derivatives: chemistry, physicochemical data, toxicity, and antitumor activities against P388 and L1210 leukemias" J. MED. CHEM. (1985), 28(8), 1079-88 , XP000906978
- GIANNOLA, L. I. ET AL: "Synthesis and characterization of amino acidic prodrugs of valproic acid" PHARMAZIE (1998), 53(12), 829-834 ,1998, XP000906977
- PAPANASTASSIOU, ZINON B. ET AL: "Potential carcinolytic agents. V. Enamine mustards" J. MED. CHEM. (1967), 10(4), 701-6 , XP000906981
- CHAYKOVSKY, MICHAEL ET AL: "Methotrexate analogs. 6. Replacement of glutamic acid by various amino acid esters and amines" J. MED. CHEM. (1975), 18(9), 909-12 , XP000906980
- GIANNOLA L I ET AL: "L-PYROGLUTAMYL-L-TRYPTOPHAN DERIVATIVES AS POTENTIAL DRUG CARRIERS" PHARMAZIE,DD,VEB VERLAG VOLK UND GESUNDHEIT. BERLIN, Bd. 51, Nr. 7, 1. Juli 1996 (1996-07-01), Seiten 487-489, XP000592082 ISSN: 0031-7144

## Beschreibung

Die Erfindung betrifft die Verwendung von Tryptophanylestern und deren N-Acyl-Derivaten zur Herstellüng eines Arzneimittels zur Prävention und Therapie von Erkrankungen, die durch oxidative Prozesse, sogenannten oxidativen Streß, entstehen oder von ungewünschten Oxidationen zellulärer Moleküle begleitet werden. Bekannte derartige Krankheiten umfassen sowohl degenerative Erkrankungen wie Morbus Alzheimer, Morbus Parkinson, Amyotrophe Lateralsklerose (ALS), Schlaganfall, Herzinfarkt, Artherosklerose, Katarakte als auch verschiedene Krebsformen, wie Brustkarzinom, Melanom, Gehimtumore.

Es ist bekannt, daß oxidative Prozesse bei der Pathogenese verschiedener degenerativer Erkrankungen aber auch bei der Entstehung des unkontrollierten Zellwachstums verschiedener Krebsarten eine zentrale Rolle spielen. Dabei sind Oxidantien regelmäßige Beiprodukte des zellulären Metabolismus, können jedoch Eiweisse (Protein), Fettsäuren (Lipid) und Desoxyribonukleinsäuren (DNA) schädigen, wenn sie nicht rechtzeitig und effizient in harmlose Verbindungen umgewandelt werden. Oxidative Schädigungen zentraler Funktionseinheiten der Zellen tragen ursächlich-zur Krebsentstehung (Ames, Science, Vol. 221, pp. 1256-1264, 1983), zu Herz-Kreislauf-Erkrankungen (z.B. Arteriosklerose, Herzinfarkt, siehe Parthasarathy, Annu. Rev. Med., Vol. 43, pp. 219-225, 1992), zu Immunschwäche und vor allem zu Störungen der Gehirnfunktionen, z.B. Gedächtnisstörungen, Durchblutungsstörungen (Coyle und Puttfarcken, Science, Vol. 262, pp. 689-695, 1993), zu neurodegenerativen Erkrankungen und Gehimzelltod, (z.B. Morbus Alzheimer, Morbus Parkinson, Schlaganfall, ALS) (Coyle und Puttfarcken, Science, Vol. 262, pp. 689-695, 1993; Beal, Ann. Neurol., Vol. 38, pp. 357-366, 1995), sowie allgemein zu verschiedenen degenerativen Alterungserscheinungen bei (zur Übersicht siehe Ames, Proc. Natl. Acad. Sci. USA, Vol. 90, pp. 7915-7922, 1993). Die Oxidation von Makromolekülen spielt auch bei der Entstehung der Arteriosklerose eine entscheidende Rolle. Hierbei wird das sogenannte Low-Density-Lipoprotein (LDL) in seiner oxidierten Form in den Ablagerungen der arteriosklerotischen Gefäßverengungen gefunden. Oxidiertes LDL besitzt erhöhte Aggregationseigenschaften. Ziel ist es, für eine Prävention und/oder Therapie der Arteriosklerose, die Oxidation von LDL mit Antioxidantien zu verhindern (Parthasarathy et al., Annu Rev Med, Vol. 43, pp. 219-225, 1992).

Oxidativer Stress wird auch als ein Initiator für die Entwickiung von Katarakten angesehen, u.a. da eine extensive Oxidation von Proteinen der Sehlinse mit humanen Katarakten einhergeht. Weiterhin sind die Peroxidspiegel in Kataraktgeweben erhöht (Spector, FASEB J., Vol. 9, pp. 1173-1182, 1995). Die Akkumulation von Peroxiden ist ein allgemeiner Mechanismus, durch den oxidativer Streß vermittelt wird (Ames, Science, Vol. 229, pp. 1256-1264, 1983; Behl, Progress in Neurobiology, Vol. 57, pp. 301-323, 1999). Damit stellt oxidativer Streß eine der wichtigsten Ursachen zur Entstehung verschiedenster Erkrankungen dar. Obgleich im Organismus und auf Zellebene körpereigene Antioxidationssysteme existieren, sind diese bei altersbedingten oder pathologisch gestiegenem oxidativem Milieu wie etwa während und nach einem Schlaganfall, Herzinfarkt, ALS oder bei der Alzheimer'schen Erkrankung bei weitem nicht ausreichend und führen zu den für diese Erkrankungen charakteristischen oxidativen Veränderungen.

JS 4,448,785 beschreibt die Verwendung von ungesattigien Fettsaurenamiden von Tryptophanderivaten als antiartherosklerotische Arzneimittel

In EP 0 240 352 A2 werden 5-Fluorouracil Denvate, u.a. Tryptophanylester, zur Behandlung von Krebserkrankungen offenbart.

J Med. Chem. (1985), 28, pp. 1079-1088 offenbart chemotherapeutisch wirksame Vinblastin-Derivate die u a. an Tryptophanylester gekoppelt sind.

In J. Ferment. Technol. (1976), 54(6), pp-369-373 werden antibakteriell wirksame N-Acyl-Derivate von Aminosäuren wie z.B., des Laurylesters des DL-Tryptophans beschneben.

US 5,599,947 offenbart D- und L-Tryptophanylester als antivirale Substanzen.

Generell muß der Körper natürliche Antioxidantien (z.B. Vitamin C und E) mit der Nahrung (z.B. Früchte und Gemüse) zuführen oder selbst synthetisieren (z.B. Glutathion), um Zellen vor oxidativem Schaden und langfristig vor oxidativen Erkrankungen zu schützen. Diese natürliche Antioxidation ist jedoch bei pathologischen Ereignissen oder während alters-assoziierten oxidativen Ereignissen völlig unzureichend. Neue, vor allem effektivere Antioxidantien mit einfacher chemischer Struktur werden daher benötigt, die aufgrund guter Fettlöslichkeit (hohe Lipophilie) die zellulären Zielregionen oxidativer Prozesse (z.B. zelluläre Membransysteme) in hoher Konzentration erreichen.

Überraschender Weise wurden solche neuen Antioxidantien mit hoher Lipophilie unter Verwendung eines experimentellen Paradigmas zum oxidativen Zelltod mit den

Überraschender Weise wurden solche neuen Antioxidantien mit hoher Lipophilie unter Verwendung eines experimentellen Paradigmas zum oxidativen Zelltod mit den hier vorgestellen Tryptophanylestern und deren N-Acyl-Derivaten jetzt erstmals gefunden.

Obgleich seit langem bekannt ist, daß oxidative Prozesse die lebenswichtigen Strukturen der Zelle schädigen und pathologische Veränderungen hervorrufen können, gibt es in Ermangelung geeigneter potenter Antioxidantien bis heute nur wenig effektive und zur Zeit noch äußerst unbefriedigende Therapieansätze, die zur Verhinderung derartiger pathologischer Prozesse gezielt eingesetzt werden können (Behl, Progreess in Neurobiology, Vol. 57, pp. 301-323, 1999).
Als potentielle präventive und therapeutische Antioxidantien sind neben dem Vitamin E und C z.Zt. nur polyzyklische Phenolverbindungen bekannt. Ausgehend vom weiblichen Sexualhormon Östrogen wurden solche polyzyklischen Phenolverbindungen und lipophile aromatische Alkoholverbindungen als antioxidativ neuroprotektiv wirksame Strukturen identifiziert und vorgeschlagen (*Behl et al., Biochem. Biophys. Res. Commun., Vol. 216, pp. 473-482, 1995; Behl et al., Molecular Pharmacology, Vol. 51, pp. 535-541, 1997; Moosmann et al., FEBS Letters, Vol. 413, pp. 467-472, 1997*).

Es müssen aber entscheidende Nachteile beim Einsatz von polyzyklischen Phenolverbindungen und Östrogenderivaten in Betracht gezogen werden. Solche entscheidenden Nachteile des potentiellen Antioxidans Östrogen, verschiedener Östrogenderivate und polyzyklischer Phenolverbindungen, welche einen gezielten therapeutischen Einsatz verhindern, sind:
1. Die geringe Wirkeffizienz, d.h. für einen therapeutischen Einsatz wären vergleichsweise hohe Konzentrationen der Substanzen nötig. Im experimentellen Ansatz sind für einen 50%igen Zellschutz vor Oxidationen EC₅₀- Werte im mikromolaren Bereich notwendig (Goodmann et al., J. Neurochem., Vol. 66, pp. 1836-1844, 1996; Behl et al., Molecular Pharmacology, Vol. 51, pp. 535-541, 1997; Moosmann et al., FEBS Letters, Vol. 131, pp. 467-472, 1997).
2. Die polyzyklischen Phenolverbindungen besitzen eine hohe Affinität zum Östrogenrezeptor, was zu völlig unerwünschten feminisierenden Nebenwirkungen führt (Katzenellenbogen, Environmental Health Perspectives, Vol. 103, pp. 99-101, 1995; Miksicek, Proc. Society for Exp. Biol. & Med., Vol. 208, pp. 44-50, 1995; Ojasoo et al., Steroids, Vol. 60, pp. 458-469, 1995; Cook et al., Regulatory Toxicology & Pharmacology, Vol. 26, pp. 60-68, 1997; Richardmeier, et al. General & Comparative Endocrinology, Vol. 100, pp. 314-326, 1995).
3. Die Aktivierung von Östrogenrezeptoren durch Östrogene bzw. ÖstrogenDerivate und Phenole wird mit der Entstehung bzw. der Verstärkung verschiedener Brustkrebsarten ursächlich in Verbindung gebracht (Biswas, Molecular Medicine, Vol. 4, pp. 454-467, 1998; White und Parker, Endocrine-Related Cancer, Vol. 5, pp. 1-14, 1998; Khan, J. Natl. Cancer Inst., Vol. 90, pp. 37-42, 1998; Santodonato, Chemosphere, Vol. 34, pp. 835-848, 1997).
4. Ein potentieller Einsatz dieser Substanzen ist als auf neurodegenerative Prozesse beschränkt beschrieben (z.B. Simpkins und Gordon, WO 97/03661).
5. Phenolische Verbindungen liegen in vivo oft in Gleichgewicht mit derivatisierten Formen vor (z.B. glykosylierte und acetylierte Derivate), so daß keine hohe Konzentration am Zielort, z.B. im Gehirn, erreichbar ist (Forth, Henschler, Rummel, Starke: Pharmakologie und Toxikologie-Lehrbuch, BI-Wissenschaftsverlag, 1992; Gonzalez, Med: Hypotheses, Vol. 32, pp. 107-110, 1990; Martucci und Fishman, Pharmacol Therapy, Vol. 57, pp. 237-257, 1993; Zhu und Conney, Carcinogenesis, Vol. 19, pp. 1-27, 1998).
6. Die oben genannten Verbindungen sind zum Redox-Cycling fähig. Das Redox-Cycling ist ein prooxidativer Effekt, der für pathologische Prozesse bei der Parkinson'schen Erkrankung (Ebadi et al., Progress in Neurobiology, Vol. 48, pp. 1-19, 1996) verantwortlich gemacht wird und generell dem antioxidativen Konzept entgegensteht, d.h. Metaboliten der phenolischen Antioxidantien sind Prooxidantien (Ames, Science, Vol. 221, pp. 1256-1264, 1983; Thompson, Chem Res Toxicol, Vol. 8, pp. 55-60, 1995; Gut, Environm Health Perspect, Vol. 104 Suppl. 6, pp. 1211-1218, 1996).
7. Verschiedene Phenole verhalten sich unter speziellen Bedingungen (Phenol/Sauerstoff-Partialdruckverhältnis; Anwesenheit von Kupfer oder anderen Schwermetallionen) selbst prooxidativ (Yamashita, Chem Res Toxicol, Vol. 11, pp. 855-862, 1998). Es ist wahrscheinlich, daß solche Bedingungen auch in vivo vorliegen, was den Einsatz von Phenolen als Antioxidantien zusätzlich in Frage stellt.

Diese entscheidenden Nachteile erschwerden den Einsatz dieser Substanzen für eine erfolgreiche Prävention und Therapie bzw. können auch (wie etwa für einen Einsatz im männlichen Organismus) kontraindiziert sein. Aufgabe der vorliegenden Erfindung ist es somit, ein Arzneimittel und dessen Verwendung bereitzustellen, das gezielt zur Prävention und vor allem Therapie von oxidativen pathologischen Veränderungen eingesetzt werden kann, ohne die vorstehend genannten Nebenwirkungen hervorzurufen.

Gegenstand der Erfindung ist somit die Verwendung von Tryptophanylestern und deren N-Acyl-Derivaten gemäß Ansprüchen 1 bis 11 und ein Arzneimittel gemäß Anspruch 12.

Lipophile Tryptophanylester und deren N-Acyl-Derivate wirken in signifikant geringeren EC₅₀-Konzentrationen (im mittleren nanomolaren Bereich, also bis zu 100 mal besser als polyzyklische Phenolverbindungen) antioxidativ, was pharmakologisch gewünscht ist und das Erreichen adäquater Wirkkonzentrationen der Substanzen am Zielort (z.B. Gehim) aus pharmakologischer Sicht ermöglicht. Vor allem besitzen solche lipophilen Tryptophanylester und deren N-Acyl-Derivate keine Affinität zu Östrogenrezeptoren und somit keine Östrogenrezeptor-aktivierende Wirkung.

Es wurde nun unerwarterterweise gefunden, daß die oben genannten Tryptophanylester und deren N-Acyl-Derivate eine ausgeprägte antioxidative und zytoprotektive Wirkung gegen krankheitsrelevante oxidative pathologische Prozesse entfalten.

Bei fast allen oxidativen Prozessen in der Zelle sind zumindest an einem entscheidenden Reaktionsschritt elektrophile Spezies mit ungepaarten Elektronen, also freie Radikale, beteiligt. Da solche Reaktionen kinetisch nicht gehemmt sind (je nach der Art des Elektrophils; Triplettsauerstoff-Reaktionen sind kinetisch stark gehemmt, deshalb verursacht Triplettsauerstoff (Sauerstoff im Normalzustand) keinen oder nur geringen oxidativen Streß; Superoxidradikalanion-Reaktionen sind kinetisch nicht gehemmt, sie verursachen starken oxidativen Streß. Da diese mit hohen Geschwindigkeitskonstanten ablaufen, sind zur Verhinderung solcher oxidativer Prozesse kleine, gut diffundierende Moleküle notwendig, die vor Ort in den Reaktionsmechanismus der potentiell schädlichen Elektrophile eingreifen und einen alternativen Reaktionsweg bereitstellen. Dieser alternative Reaktionsweg könnte im Falle der lipophilen Tryptophanylester und deren N-Acyl-Derivate in der energetisch favorisierten und kinetisch schnellen Bereitstellung eines Wasserstoffradikals (H*) bestehen, welches mit dem Elektrophil (E*) reagiert und somit zu einer chemisch inerten Valenz mit gepaartem Elektronenpaar (E-H) führt. Dabei entstände zwar ein neues Antioxidansradikal (A*), doch dieses ist aufgrund des direkt benachbarten aromatischen Systems sowie des elektronenreichen Stickstoffatoms energetisch so inert, daß es nicht mit den üblichen zellulären Nucleophilen zu reagieren in der Lage ist. Seine Lebensdauer ist also groß genug, um durch Diffusion eines der speziellen zelleigenen Ein-Elektronen-Reduktionssysteme wie reduziertes Nicotin-Adenin-Dinucleotid (NADH) zu erreichen und um von diesem recycelt zu werden. Dies ist eine mögliche Erklärung zur antioxidativen Wirkung der erfindungsgemäßen Tryptophanylester und deren N-Acyl-Derivate, die nicht zu einer Festlegung führen soll.

Der entscheidende Vorteil des Einsatzes von Tryptophanylestern und deren N-Acyl-Derivaten liegt neben der Vermeidung von östrogenartigen Nebenwirkungen und der Vermeidung von metabolischen Problemen (siehe oben, Punkte 1-7) in dem ausschließlich bei Verwendung von Stickstoff möglichen Aufbau eines Moleküls, das zugleich ein labiles Wasserstoffatom trägt und dessen Wasserstoffträger (Stickstoff) von zwei aromatischen Systemen stabilisiert wird (Dreiwertigkeit des Stickstoffs). Die Verwendung von Sauerstoff als Wasserstoffträger, wie sie in vielen phenolischen Antioxidantien wie Vitamin E und Östrogen realisiert ist, erlaubt nur ein aromatisches System neben dem labilen Wasserstoffatom. Die Überlegenheit der lipophilen Tryptophanylester und deren N-Acyl-Derivaten ist also prinzipieller chemischer Natur und findet in der hier überraschender Weise gefundenen 100fach effektiveren Wirkung gegenüber den Phenolen ihren Ausdruck.

Erfindungsgemäße Tryptophanylester und deren N-Acyl-Derivate sind.

Tryptophanylester und deren N-Acyl-Denvate mit der unten dargestellten Formel (I) 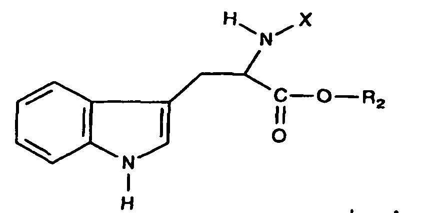 wobei X C(O)R1 oder H ist und R1 ein gesättigter C₂-C₈ Kohlenwasserstoffrest ist und R2 ein gesättigte oder ungesättigte C₂-C₁₈ Kohlenwasserstoffreste sind und das Tryptophangerüst in D- und L-Konfiguration vorliegen kann, zeigen eine ausgeprägte zytoprotektive und antioxidative Wirkung gegen oxidative und degenerative pathologische Prozesse. Hierbei sind Tryptophanoctylester, und N-Dodecanoyl-tryptophanethylester besonders bevorzugte Ausführungsformen.

Es sind eingeschlossen alle Substanzen, die in einer Pro-Form verabreicht werden und im Körper in eine der wirksamen, wie oben definierten Strukturen metabolisiert werden.

Die erfindungsgemäß verwendeten Verbindungen sind bekannt oder können in dem Fachmann bekannter Weise hergestellt werden (Beilsteins Handbuch der Organischen Chemie, Haupt- und Ergänzungswerke, Jahrgänge 1909-1979).

Die vorliegenden Verbindungen können auf verschiedene Arten verabreicht werden, um die gewünschte Wirkung zu erreichen. Die Verbindungen können alleine oder in der Form pharmazeutischer Zubereitungen dem behandelten Patienten entweder oral oder parenteral, zum Beispiel subcutan, intravenös, intramuskular, oder intracerebral verabreicht werden. Ebenso können sie durch Inhalation oder durch Zäpfchen verabreicht werden. Sie können aber auch auf anderer Weise, z.B. transdermal, verabreicht werden, sofern hierdurch die erforderlichen Dosierungen erreicht werden können. Die Menge der verabreichten Verbindung kann unterschiedlich sein und dabei kann es sich um eine beliebige Menge handeln, die zur Prophylaxe und/oder Therapie von oxidativen pathologischen Prozessen und/oder Krebserkrankungen dient. In Abhängigkeit vom Patienten, Schwere der Krankheit und der Art der Verabreichung kann die verabreichte Menge der Verbindung über einen weiten Bereich variieren, wobei von etwa 0,1 mg/kg bis zu etwa 10 mg/kg, normalerweise 1 bis 5 mg/kg Körpergewicht des Patienten pro Dosis bereitgestellt werden. Einheitsdosen dieser Verbindungen können zum Beispiel von etwa 10 mg bis 100 mg, normalerweise 50 bis 500 mg und vorzugsweise 250 bis 500 mg der Verbindung enthalten und können zum Beispiel 1- bis 4-mal pro Tag verabreicht werden.
Wie hier verwendet, bedeutet die Bezeichnung "Einheitsdosis" eine Form einer Einfach- oder Mehrfachdosis, die eine Menge des Wirkstoffs in Beimischung oder sonst in Verbindung mit dem Verdünnungsmittel oder dem Träger enthält, wobei die Menge so ist, daß normalerweise eine oder mehrere vorbestimmte Einheiten für eine einzelne therapeutische Verabreichung erforderlich sind. Im Fall einer Mehrfachdosisform, wie zum Beispiel Flüssigkeiten oder eingekerbte Tabletten, ist die vorbestimmte Einheit ein Bruchteil der Mehrfachdosisform, wie eine 5 ml-Menge (Teelöffel) einer Flüssigkeit oder eine Hälfte oder ein Viertel einer eingekerbten Tablette.
Einzelne erfindungsgemäße Formulierungen werden auf eine an sich in der Pharmazie weithin bekannte Art und Weise hergestellt und umfassen in der Regel mindestens einen erfindungsgemäßen Wirkstoff in Beimischung oder sonst in Verbindung mit einem pharmazeutisch verträglichen Träger oder Verdünnungsmittel dafür. Zur Herstellung dieser Formulierung wird der Wirkstoff in der Regel mit einem Träger gemischt oder mit einem Verdünnungsmitel verdünnt und in eine Kapsel, eine Gelatinekapsel, eine Tüte, ein Papier oder ein anderes Behältnis eingeschlossen oder eingekapselt. Bei einem Träger oder Verdünnungsmittel kann es sich um ein festes, halbfestes oder flüssiges Material handeln, das als Trägermittel, Excipient oder Medium für den Wirkstoff dient. Geeignete Träger oder Verdünnungsmittel sind an sich bekannt. Eine Beschreibung der Herstellung solcher Formulierung ist in Remington's Pharmaceutical Sciences, Mack Publishing Company, Easton, Pennsylvania zu finden.
Die erfindungsgemäß verwendeten Verbindungen können vorzugsweise bei der Therapie oder Prophylaxe von Morbus Alzheimer oder amyotropher Lateralsklerose alleine oder in der Kombination mit anderen Therapeutika, die eine, andere Wirkungsweise haben, verwendet werden. Zu solchen Arzneimitteln gehören, zum Beispiel zur Therapie und/oder Prophylaxe von Morbus Alzheimer Ciliary Neurotropic Factor (CNTF) und Aniracetam, Buflomedil, Cholin, Co-dergocrin, Cyclandelat, Desferrioxamin, Eptastigmin, Fampridin, Galantamin; Isoxsuprin, Lecithin, Linopirdin, Metriphonat, Naftidrofuryl, Nicergolin, Nikotin, Nimodipin, Oxiracetam, Physostigmin, Pilocarpin, Piracetam, Pramiracetam, Propentofyllin, Pyritinol, RS-86, Selegilin, Suronacrin, Tacrin, Velnacrin, und zur Therapie von amyotropher Lateralsklerose Somatomedine, Protirelin, Immunoglobuline und Immunsupressiva.

Die oben genannten Verbindungen können in dem Fachmann bekannter Weise, gegebenfalls mit einem üblichen Trägermaterial zu einem Arzneimittel formuliert werden.

Die Verabreichung der oben genannten Verbindungen als Arzneimittel kann oral, rektal, intravenös, intramuskulär, intracerebral, parenteral oder inhalatorisch erfolgen. Sie können aber auch auf anderer Weise, z.B. transdermal, verabreicht werden, sofern hierdurch die erforderlichen Dosierungen erreicht werden können.

Die Figuren zeigen:
- Figur 1:: Darstellung der Wirkungscharakteristik von Tryptophanoctylester
- Figur 2:: Darstellung der Wirkungscharakteristik von N-Oleoyl-tryptophanethylester
- Figur 3:: Darstellung der Wirkungscharakteristik von N-Dodecanoyl-tryptophanethylester

Die Wirksamkeit der erfindungsgemäßen Verwendung von Tryptophanylestern und deren N-Acyl-Derivaten wird durch die nachstehenden Versuche belegt.

### Beispiel 1:

Als experimentelles System wurden Nervenzellen (clonale hippocampale Zelle aus der Maus, sogenannte HT22-Zellen; *Morimoto und Koshland, Neuron, Vol. 5, pp. 875-880, 1990;* und clonale menschliche Neuroblastomzellen, sogenannte SK-N-MC-Zellen; ATCC# HTB10) und murine Fibroblastenzellen, sogenannte NIH3T3-Zellen (ATCC# CRL1658), in der Zellkultur unter oxidativen Streß gesetzt. Oxidativer Streß wurde durch anerkannte und in der Literatur vielfach verwendete Oxidantien, die innerhalb kurzer Zeit zum Zelltod durch Peroxidation zellulärer Makromoleküle führen, ausgelöst: im Fall der Nervenzellen etwa durch die exzitatorische Aminosäure Glutamat sowie Wasserstoffperoxid und im Fall der Fibroblastenzellen nur Wasserstoffperoxid. Die Zahl der überlebenden Zellen wurde durch verschiedene standardisierte Testverfahren, vor allem durch die Messung der zellulären mitochondrialen Aktivität bestimmt. Diese Ergebnisse wurden durch die mikroskopische Zellzahlbestimmung nachfolgend bestätigt.

Zellen (z.B. HT22, SK-N-MC, NIH3T3) werden in Standard-Kulturmedium (DMEM supplementiert mit 10% fötalem Kälberserum) in Kulturschalen ausgesät. So werden die Zellen zunächst über Nacht stehen gelassen, um das quantitative Absetzen der Zellen auf die Kulturoberfläche zu gewährleisten. Die Zelldichte beim Aussäen war für HT22 10%, Für SK-N-MC 25% und für NIH 3T3 15%.

Anschließend werden die potentiellen neuroprotektiven Substanzen in verschiedenen Endkonzentrationen hinzugegeben (Dauer der Inkubation zwischen 2-4 Stunden). Anschließend werden die Zellkulturen mit den oben definierten oxidativen Stressoren inkubiert, so etwa mit Glutamat oder mit Wasserstoffperoxid in jeweils unterschiedlichen Konzentrationen. Die Konzentration der oxidativen Stressoren ist so gewählt, daß die Vitalität der nicht mit den protektiven Substanzen vorinkubierten Kontrollzellen weniger als 15% beträgt, somit für Glutamat 3 mM (HT22), für Wasserstoffperoxid 160 µM (SK-N-MC) oder 200 µM (NIH 3T3). Diese Inkubation wird ca. 20 Stunden durchgeführt, bevor 3-(4,5-Dimethylthiazol-2-yl)-2,5 diphenyl-tetrazoliumbromid (MTT) (Endkonzentration 0.5 mg/ml, von Sigma) zu den Zellen gegeben wird, um die mitochondriale Aktivität der Kulturzellen zu bestimmen. MTT wird von mitrochondrialen Enzymen der Zelle zu einem blauen Formazan-Farbstoff umgesetzt. Diese Farbreaktion wird 4 Stunden durchgeführt. Danach wird die Reaktion mit der Zugabe einer Detergenzlösung gestoppt (SDS 10%/DMF 50%, pH 4.8) und in einem Photometer bei einer Wellenlänge von 570 nm gelesen.

Das hier beschriebene Protokoll ist eine für kultivierte Nervenzellen modifizierte Version (siehe *Behl, Biochem. Biophys. Res. Comm.,* Vol. 186, pp. 944-950, 1992) des ursprünglich publizierten Protokolls (*Hansen, J. Immunol. Meth.,* Vol. 119, pp. 203-210, 1989).

Zur Bestätigung der MTT-Daten wird in einem anderen Überlebenstest nach Abschluß der Toxin-Reaktion der Farbstoff Trypan-Blau zu den Zellen gegeben. Trypan-Blau kann nur geschädigte Membranen toter Zellen penetrieren. Die tote Zelle färbt sich blau und kann unter dem Mikroskop gezählt werden. Die Anzahl der lebenden, nicht gefärbten, und der toten Zellen wird genau bestimmt. Diese experimentellen Systeme zur Entdeckung antioxidativ zytoprotektiver Substanzen sind hoch reproduzierbar einsetzbar.

Der MTT- sowie der Zellzahl-Test werden nach Standardverfahren durchgeführt (Behl, Cell, Vol. 77, *pp. 817-827, 1994*).

### Protokoll des Hirnmembrandesintegrations-Experiments:

Native Rattenhirnmembranen werden in einer durch geringe Dosen von Ascorbat (50µM) katalysierten Reaktion oxidiert (genaueres siehe bei Moosmann und Behl, PNAS 96, S. 8867-8872, 1999). Diese Reaktion ist mit dem Auftreten von elektronisch angeregten Primäroxidationsprodukten verbunden, die unter Abgabe von Photonen in den Grundzustand übergehen. Durch Messung der emittierten Einzelphotonen kann der Dekompositionsprozeß der Hirnmembranen in Echtzeit verfolgt und zeitlich integriert werden. Die Messung erfolgte sechs Stunden nach der Ascorbatzugabe mit einem single-photon-counting-Szintillationszähler der Firma Beckman.

Die Resultate zeigen, daß die Substanzen auch bereits vorgeschädigte Hirnmembranen vor weiterer Zerstörung zu schützen vermögen, dies unabhängig vom Vorhandensein endogener Schutzmechanismen wie beispielsweise aktivierter Immunzellen.

### Beispiel 2:

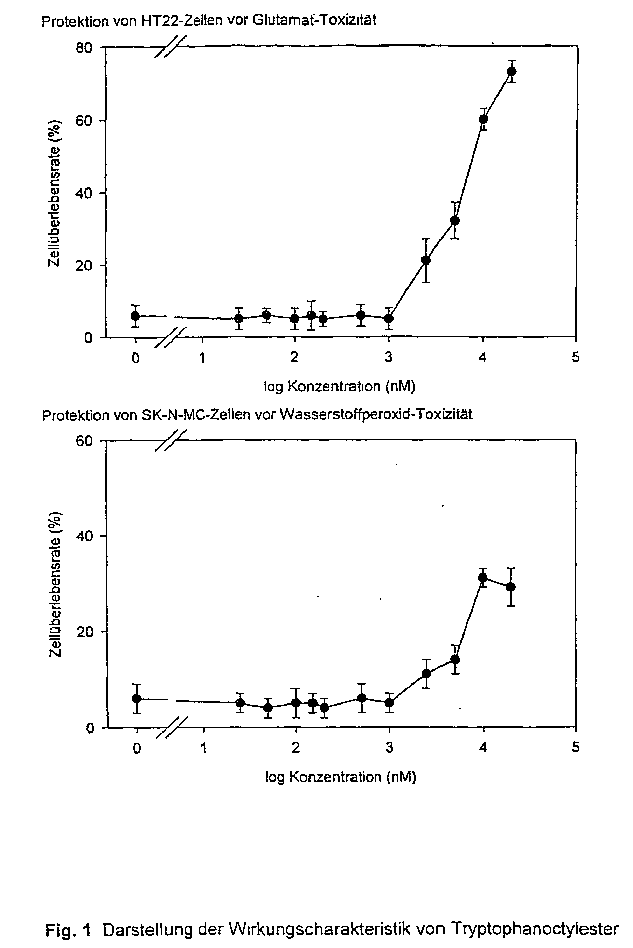

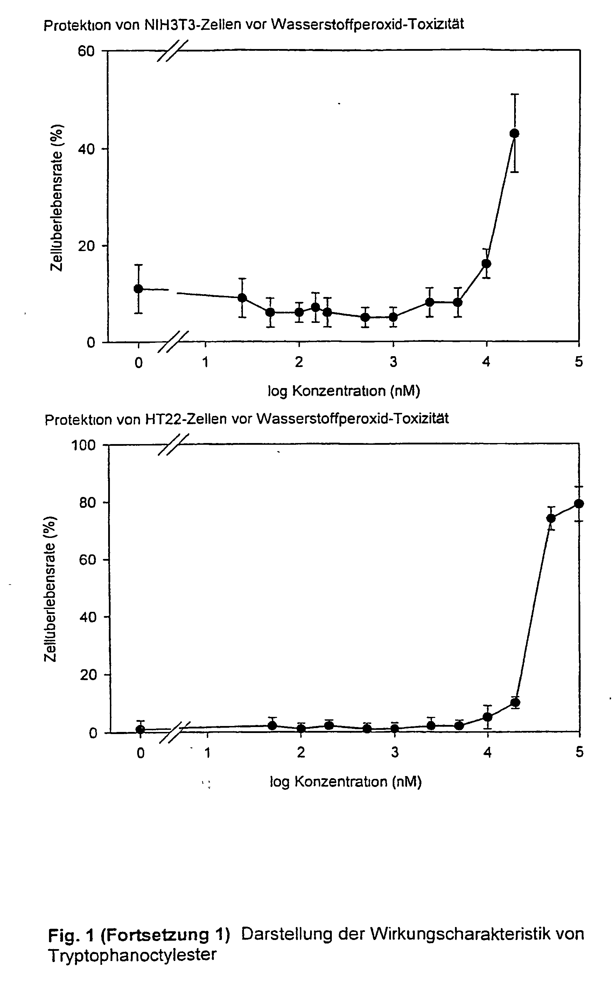

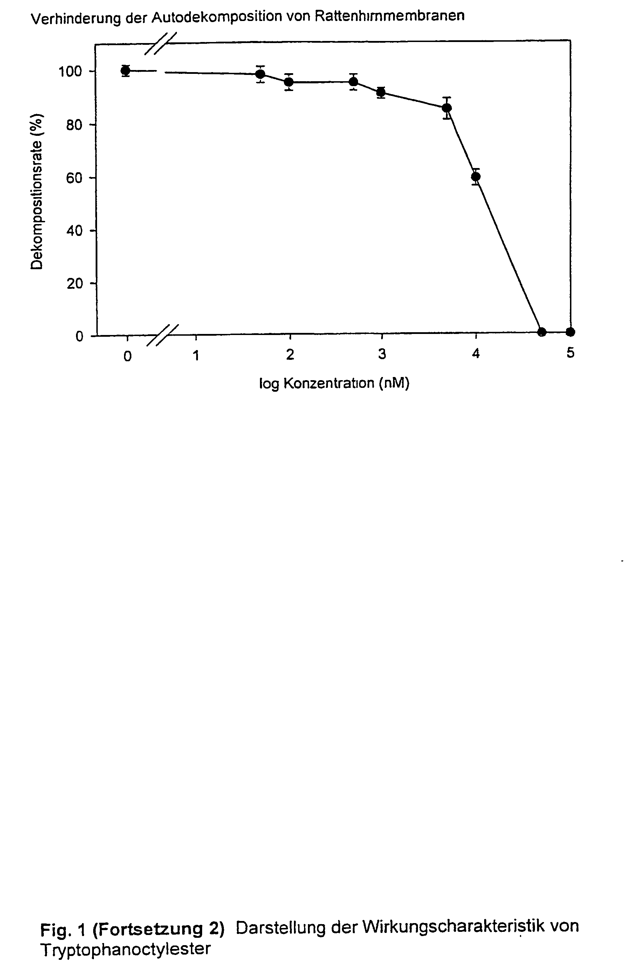

Tryptophanoctylester wurde in den Konzentrationen 25 nanomolar bis 20 mikromolar wie in Beispiel 1 beschrieben an HT22-Zellen, SK-N-MC-Zellen, NIH3T3-Zellen und Rattenhirnmembranen getestet. Das Ergebnis ist in Figur 1 dargestellt.

### Beispiel 3: (Referenzbeispiel)

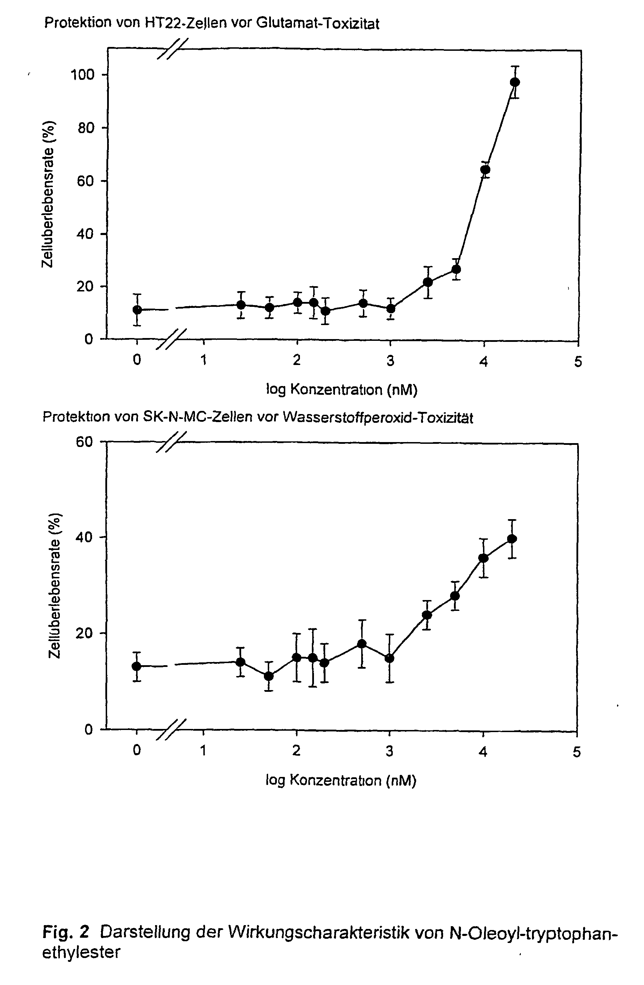

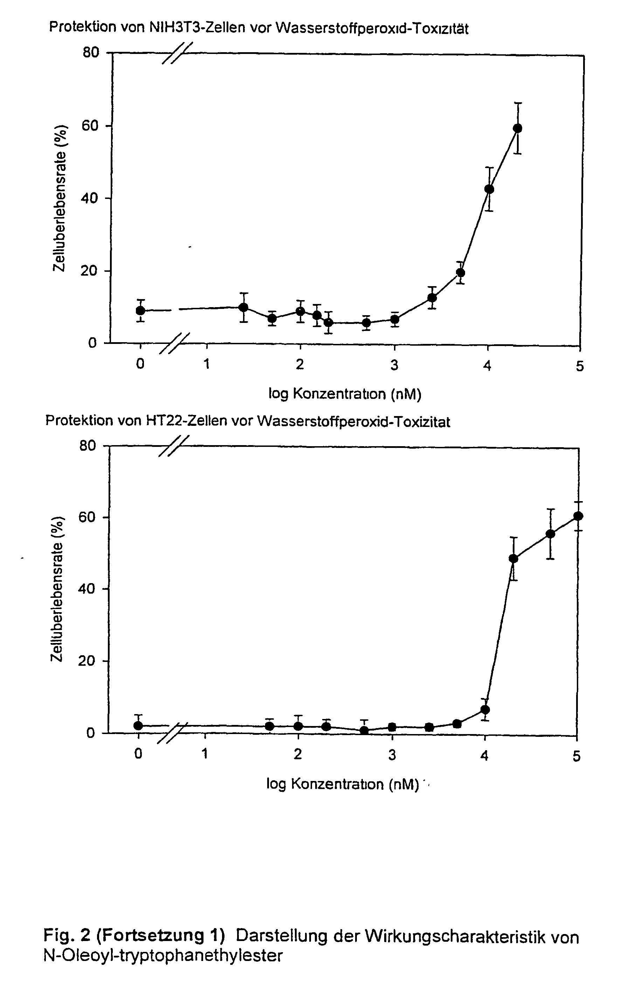

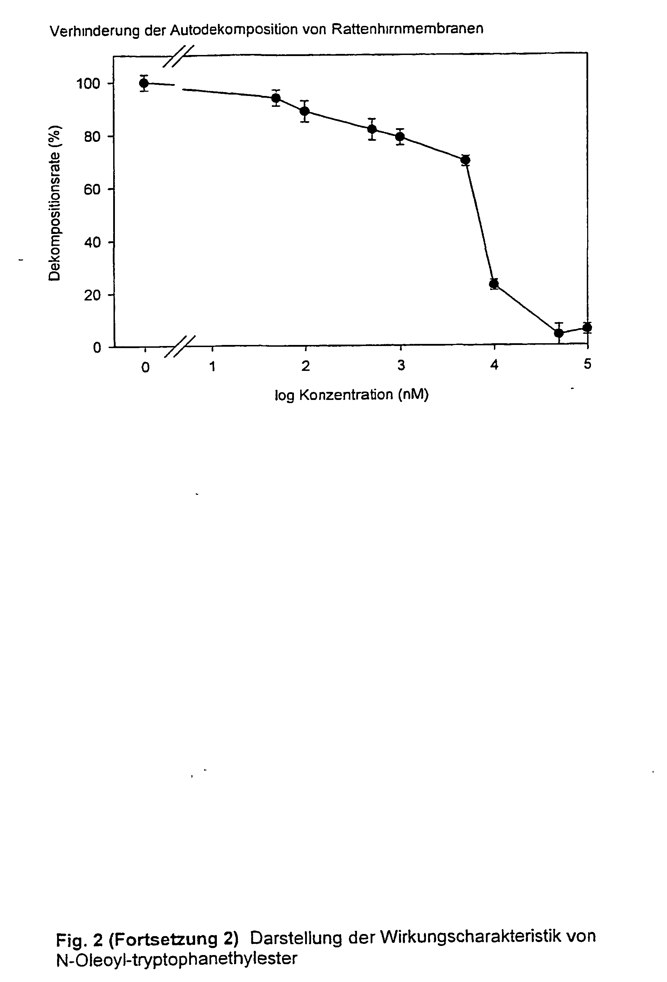

N-Oleoyl-tryptophanethylester wurde in den Konzentrationen 25 nanomolar bis 20 mikromolar wie in Beispiel 1 beschrieben an HT22-Zellen, SK-N-MC-Zellen, NIH3T3-Zellen und an Rattenhimmembranen getestet. Das Ergebnis ist in Figur 2 gezeigt.

### Beispiel 4:

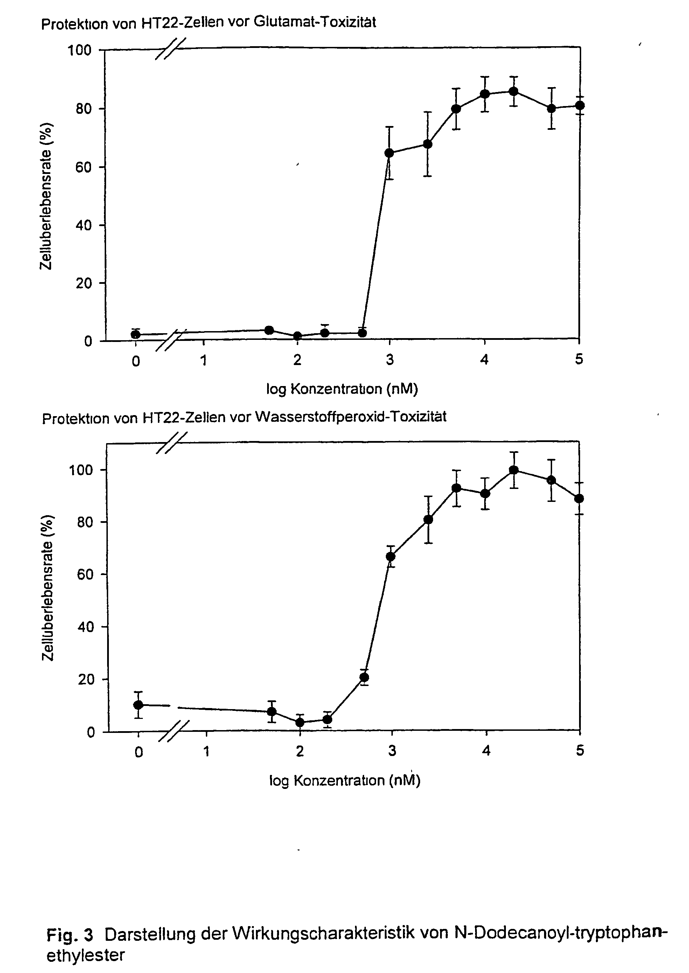

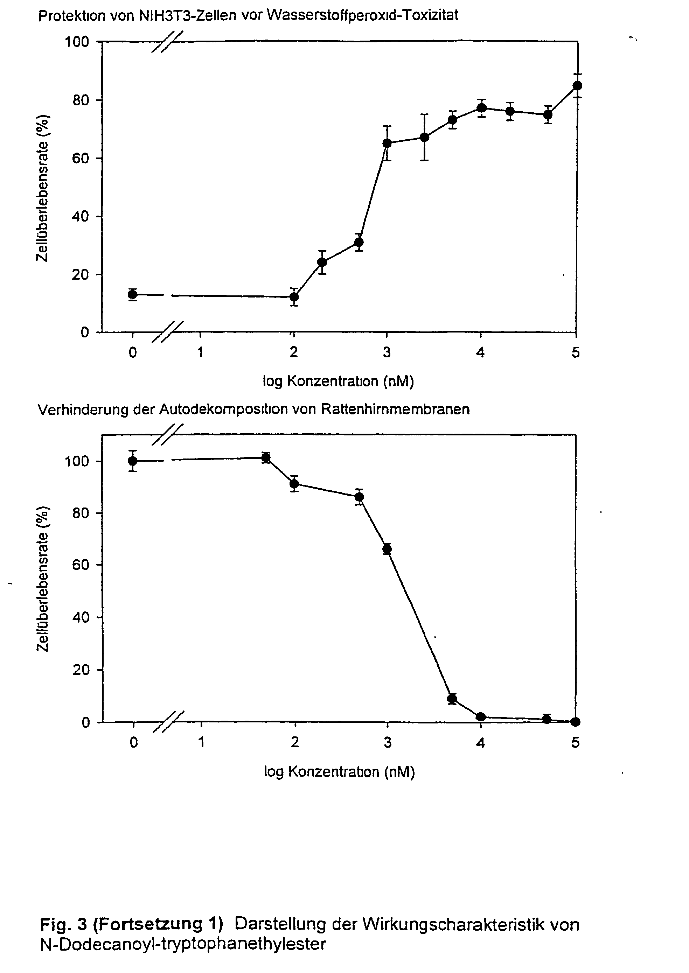

N-Dodecanoyl-tryptophanethylester wurde in den Konzentrationen 25 nanomolar bis 100 mikromolar wie in Beispiel 1 beschrieben an HT22-Zellen, NIH3T3-Zellen und an Rattenhimmembranen getestet. Das Ergebnis ist in Figur 3 gezeigt.

## Patentansprüche

1. Verwendung eines D- oder L-Tryptophanylesters oder dessen N-Acyl-Derivates mit der Formel (I) 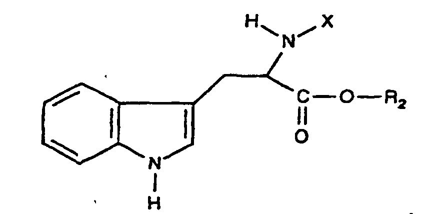 wobei X C(O)R1 oder H ist und R1 ein gesättigter C₂-C₁₈ kohlenwasserstoffrest ist und R2 ein gesättigter oder ungesättigter C₂-C₁₈ Kohlenwasserstoffrest ist zur Herstellung eines Arzneimittels zur Prophylaxe und Therapie von oxidativen pathologischen Prozessen bei degenerativen Erkrankungen und/oder Krebserkrankungen.

2. Verwendung gemäß Anspruch 1, wobei der D- oder L-Tryptophanylester oder sein N-Acyl-Derivat folgende Substanz ist:
Tryptophanoctylester, Tryptophandodecylester, Tryptophanstearylester, Tryptophanpalmitylester, Tryptophanoleylester, N-Acetyl-tryptophanoctylester, N-Acetyl-tryptophandodecylester, N-Acetyl-tryptophanstearylester, N-Acetyl-tryptophanpalmitylester, N-Acetyl-tryptophanoleylester, N-Dodecanoyl-tryptophanoctylester, N-Dodecanoyl-tryptophandodecylester, N-Dodecanoyl-tryptophanstearylester, N-Dodecanoyl-tryptophanpalmitylester, N-Dodecanoyl-tryptophanoleylester, N-Acetyl-tryptophanethylester, N-Hexoyl-tryptophanethylester, N-Octoyl-tryptophanethylester, N-Dodecanoyl-tryptophanethylester, N-Stearoyl-tryptophanethylester, oder N-Palmitoyl-tryptophanethylester.

3. Verwendung gemäß Anspruch 1, wobei der D- oder L-Tryptophanethylester oder sein N-Acetyl-Derivat Tryptophanoctylester, oder N-Dodecanoyl-tryptophanethylester ist

4. Verwendung gemäß Anspruch 1-3 zur Behandlung und/oder Prophylaxe von neurodegenerativen Erkrankungen.

5. Verwendung gemäß Anspruch 4, wobei die neurodegenerative Erkrankung Morbus Alzheimer, Morbus Parkinson, Schlaganfall oder Arnyotrophe Lateralsklerose ist.

6. Verwendung gemäß Anspruch 1-3 zur Behandlung und/oder Prophylaxe von Katarakten.

7. Verwendung gemäß Anspruch 6, wobei der Katarakt Cataracta senilis, Cataracta juvenilis, Cataracta diabetica oder Cataracta complicata ist.

8. Verwendung gemäß Anspruch 1-3 zur Behandlung und/oder Prophylaxe von neoplastischen Erkrankungen.

9. Verwendung gemäß Anspruch 8, wobei die neoplastische Erkrankung das Prostatakarzinom, das Mammakarzinom, das Leberkarzinom, das Nierenkarzinom, das Dickdarmkarzinom, das Magenkarzinom oder das Lungen-(Bronchial-)karzinom ist.

10. Verwendung gemäß Anspruch 1-3 zur Behandlung und/oder Prophylaxe von Herz-Kreislauferkrankungen.

11. Verwendung gemäß Anspruch 10, wobei die Herz-Kretsfauferkrankung Arteriosklerose oder Herzinfarkt ist.

12. Arzneimittel, umfassend einen D- oder L- Tryptophanylester oder dessen N-Acylderivat nach einem der Ansprüche 1-3 zur Prophylaxe und/oder Therapie von oxidativen pathologischen Prozessen bei degenerativen und/oder Krebserkrankungen und gegebenenfalls einen pharmazeutisch verträglichen Träger, alleine oder in Kombination mit anderen antioxidativ wirksamen oder bei degenerativen und/oder Krebserkrankungen verwendeten Arzneimitteln.

## Claims

1. Use of a D- or L-tryptophanyl ester or its N-acyl derivative with the formula (I) 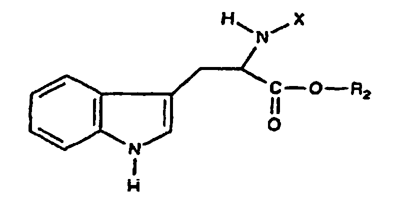 wherein X is C(O)R1 or H and R1 is a saturated C₂-C₁₈ hydrocarbon group and R2 is a saturated or unsaturated C₂-C₁₈ hydrocarbon group, for the preparation of a pharmaceutical composition for prophylaxis or therapy of oxidative pathological processes in the case of degenerative diseases and/or cancer diseases.

2. Use according to claim 1, wherein the D- or L-tryptophanyl ester or its N-acyl derivative is the following substance:
tryptophan octyl ester, tryptophan dodecyl ester, tryptophan stearyl ester, tryptophan palmityl ester, tryptophan oleyl ester, N-acetyl tryptophan octyl ester, N-acetyl tryptophan dodecyl ester, N-acetyl tryptophan stearyl ester, N-acetyl tryptophan palmityl ester, N-acetyl tryptophan oleyl ester, N-dodecanoyl-tryptophan octyl ester, N-dodecanoyl-tryptophan dodecyl ester, N-dodecanoyl-tryptophan stearyl ester, N-dodecanoyl-tryptophan palmityl ester, N-dodecanoyl-tryptophan oleyl ester, N-acetyl tryptophan ethyl ester, N-hexoyl-tryptophan ethyl ester, N-octoyl-tryptophan ethyl ester, N-dodecanoyl-tryptophan ethyl ester, N-stearoyl-tryptophan ethyl ester or N-palmitoyl-tryptophan ethyl ester.

3. Use according to claim 1, wherein the D- or L-tryptophan ethyl ester or its N-acetyl derivative is tryptophan octyl ester or N-dodecanoyl-tryptophan ethyl ester.

4. Use according to claim 1 to 3 for the treatment and/or prophylaxis of neurodegenerative diseases.

5. Use according to claim 4, wherein the neurodegenerative disease is Morbus Alzheimer, Morbus Parkinson, stroke or amyotrophic lateral sclerosis.

6. Use according to claims 1 to 3 for the treatment and/or prophylaxis of cataracts.

7. Use according to claim 6, wherein the cataract is Cataracta senilis, Cataracta juvenilis, Cataracta diabetica or Cataracta complicata.

8. Use according to claims 1 to 3 for the treatment and/or prophylaxis of neoplastic diseases.

9. Use according to claim 8, wherein the neoplastic disease is the prostate carcinoma, the mammary carcinoma, the liver carcinoma, carcinoma of kidney, colon carcinoma, gastric carcinoma or the lung (bronchial) carcinoma.

10. Use according to claims 1 to 3 for the treatment and/or prophylaxis of cardiovascular diseases.

11. Use according to claim 10, wherein the cardiovascular disease is arteriosclerosis or cardiac infarct.

12. Pharmaceutical composition comprising a D- or L-tryptophanyl ester or its N-acyl derivative according to one of the claims 1 to 3 for the prophylaxis and/or therapy of oxidative pathological processes with degenerative and/or cancer diseases and, optionally, a pharmaceutically acceptable carrier, alone or in combination with other pharmaceutical compositions which have an anti-oxidative effect or which are used in the case of degenerative and/or cancer diseases.

## Revendications

1. Utilisation d'un ester de D ou L-tryptophane ou de son dérivé n-acylé répondant à la formule (I) : 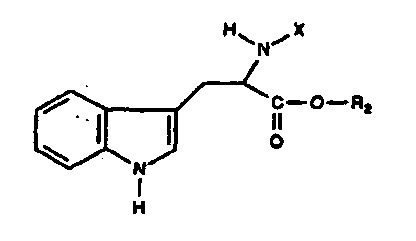 dans laquelle X représente C(O)R1 ou H et R1 représente un reste hydrocarboné en C₂ C₁₈ saturé et R2 représente un reste hydrocarboné en C₂ - C₁₈ saturé ou insaturé,
pour la préparation d'un médicament destiné à la prophylaxie et à la thérapie de processus pathologiques oxydatifs dans le cas des maladies dégénératives et/ou des maladies de nature cancéreuse.

2. Utilisation, selon la revendication 1, dans laquelle l'ester de D ou L-tryptophane ou son dérivé N-acylé est l'une quelconque des substances suivantes :
ester octylique de tryptophane, ester dodécylique de tryptophane, ester stéarique de tryptophane, ester palmitique de tryptophane, ester oléique de tryptophane, ester octylique de N-acétyl-tryptophane, ester dodécylique de N-acétyl-tryptophane, ester stéarique de N-acétyl-tryptophane, ester palmitique de N-acétyl-tryptophane, ester oléique de N-acétyl-tryptophane, ester octylique de N-dodécanoyl- tryptophane, ester dodécylique de N-dodécanoyl- tryptophane, ester stéarique de N-dodécanoyl- tryptophane, ester palmitique de dodécanoyl-tryptophane, ester oléique de N-dodécanoyl- tryptophane, ester éthylique de N-acétyl- tryptophane, ester éthylique de N-hexoyl- tryptophane, ester éthylique de N-octyl-tryptophane, ester éthylique de N-dodécanoyl-tryptophane, ester éthylique de N-stéaryl-tryptophane, ou ester éthylique de N-palmitoyl-tryptophane.

3. Utilisation selon la revendication 1, dans laquelle l'ester éthylique de D- ou L- tryptophane, ou son dérivé N-acétylé est l'ester octylique de tryptophane, ou l'ester éthylique de N-dodécanoyl-tryptophane.

4. Utilisation selon l'une quelconque des revendications 1 à 3 pour le traitement et/ou la prophylaxie de maladies neuro-dégénératives.

5. Utilisation selon la revendication 4, dans laquelle la maladie neuro-dégénérative est la maladie d'Alzheimer, la maladie de Parkinson, l'attaque d'apoplexie ou la sclérose latérale amyotropique.

6. Utilisation selon l'une quelconque des revendications 1 à 3 pour le traitement et/ou la prophylaxie de cataractes.

7. Utilisation selon la revendication 6, dans laquelle la cataracte est la cataracte sénile, la cataracte juvenilis, la cataracte diabétique ou la cataracte compliquée.

8. Utilisation selon l'une quelconque des revendications 1 à 3 pour le traitement et/ou la prophylaxie de maladies néoplasiques.

9. Utilisation selon la revendication 8, dans laquelle la maladie néoplasique est le cancer de la prostate, le cancer du sein, le cancer du foie, le cancer du rein, le cancer du colon, le cancer de l'estomac ou le cancer du poumon (des bronches).

10. Utilisation selon l'une quelconque des revendications 1 à 3 pour le traitement et/ou la prophylaxie de maladies cardio-vasculaires.

11. Utilisation de la revendication 10, dans laquelle la maladie cardio-vasculaire est l'artériosclérose ou l'infarctus du myocarde.

12. Médicament comprenant un ester de D- ou L-tryptophane ou son dérivé N-acylé selon l'une quelconque des revendications 1 à 3 pour la prophylaxie et/ou la thérapie de processus pathologiques oxydatifs dans le cas des maladies dégénératives et/ou cancéreuses et, le cas échéant, un support pharmaceutiquement compatible, seul ou en combinaison avec d'autres médicaments à effet anti-oxydatif ou utilisés dans le cas des maladies dégénératives et/ou de nature cancéreuse.
